# EUROPÄISCHE PATENTSCHRIFT

(11) **EP 1 582 575 B1**
(45) Veröffentlichungstag und Bekanntmachung des Hinweises auf die Patenterteilung: **22.11.2006**
(21) Anmeldenummer: 05101762.2
(22) Anmeldetag: 08.03.2005
(51) Int. Cl.: C09J 133/02, C09J 7/02

(54) **Haftklebeband für medizinische Diagnosestreifen**
Adhesive strips for medical diagnostic strips
Bande adhesive a usage bande diagnostique medical

(30) Priorität: 18.03.2004 DE 102004013699
(43) Veröffentlichungstag der Anmeldung: 05.10.2005
(73) Patentinhaber: tesa AG, 20253 Hamburg (DE)
(72) Erfinder: Neubert, Ingo, 22850, Norderstedt (DE); BUNDE, Bernd, 21641, Apensen (DE)

(56) Entgegenhaltungen:
- EP-A- 0 699 726
- DE-A1- 19 913 719

## Beschreibung

Die vorliegende Erfindung betrifft ein einseitiges oder doppelseitiges Haftklebeband, das für den Aufbau von medizinischen Diagnosestreifen für biologische Flüssigkeiten verwendet werden kann.

In der modernen Medizindiagnose werden für immer mehr analytische Teststreifen die so genannten Diagnoseteststreifen verwendet. Mit diesen kann zum Beispiel der Gehalt an Glukose, Cholesterol, Proteinen, Ketonen, Phenylalanin oder Enzymen in biologischen Flüssigkeiten wie Blut, Speichel und Urin bestimmt werden.

Am häufigsten anzutreffen ist die Bestimmung und Überprüfung des Blutzuckergehaltes bei Diabetikern. Weltweit leiden etwa 175 Millionen Menschen an Diabetes mellitus Typ 1 und Typ 2. Die Tendenz dieser Erkrankung ist steigend.
Bei dieser nicht heilbaren Krankheit überprüfen viele Kranken bis zu 5-mal täglich ihren Blutzuckergehalt, um die Dosierung der Medikamentation (Insulin) sowie die Nahrungsmitteleinnahme optimal aufeinander anzupassen, denn im Falle eines zu hohen Blutzuckergehalts muss mit gesundheitlichen Schäden gerechnet werden. Bisher waren die Diabetiker auf die Unterstützung von medizinischem Personal angewiesen, um den Blutzuckergehalt zu bestimmen. Um die Kontrolle des Blutzuckergehaltes so einfach wie möglich zu gestalten, wurde ein Test entwickelt, der es dem Kranken erlaubt, seinen Blutzuckergehalt, auch ohne auf medizinisches Personal angewiesen zu sein, mit geringsten Aufwand selbst zu bestimmen.
Zur Bestimmung des Blutzuckergehalts muss der Proband einen Bluttropfen auf einen Diagnoseteststreifen geben. Der Diagnoseteststreifen befindet sich dabei in einem Lesebeziehungsweise Auswertegerät. Nach einer Reaktions- oder Ansprechzeit wird auf den Auswertegerät der aktuelle Blutzuckergehalt angezeigt. Entsprechende Lese- beziehungsweise Auswertegeräte sind zum Beispiel in der US 5,304,468 A, der EP 1 225 448 A1 und der WO 03/08091 A1 beschrieben.
Mit diesem Test kann den vielen Diabetikern ein Stück Lebensqualität gegeben werden, da sie unabhängiger von Krankenhäusern und Arztpraxen sind. Darüber hinaus ist eine genauere Medikamentendosierung möglich.

Eines der ersten Patente auf dem technischen Feld der Teststreifen ist bereits 1964 erschienen. In US 1,073,596 A werden ein Diagnosetest und die Teststreifen zur Analysierung biologischer Körperflüssigkeiten speziell zur Blutzuckerbestimmung beschrieben. Der Diagnosetest funktioniert über die Bestimmung einer Farbänderung, die durch eine Enzymreaktion ausgelöst wird. Zum Aufbau des Teststreifens wird bereits die Verwendung eines Haushaltsklebers oder des Klebebandes Scotch® 464 beschrieben.

Die Bestimmung einer Konzentrationsänderung eines Farbstoffes (Kolorimetrische Methode) ist auch heute noch ein verwendetes Verfahren bei der Blutzuckerbestimmung mittels Diagnoseteststreifen. Dabei reagiert das Enzym Glukose-Oxidase/Peroxidase mit dem Blutzucker. Das entstehende Wasserstoffperoxid reagiert anschließend mit dem Indikator zum Beispiel O-Tolidine, was zu einer Farbreaktion führt. Diese Farbveränderung kann durch kolorimetrische Methoden verfolgt werden. Der Grad der Verfärbung ist direkt proportional zur Blutzuckerkonzentration. Das Enzym befindet sich hier auf einem Gewebe.
Dieses Verfahren wird zum Beispiel in EP 0 451 981 A1 und WO 93/03673 A1 beschrieben.

Die moderne Entwicklung der Diagnoseteststreifen zielt auf eine Verkürzung der Messzeit zwischen der Blutaufgabe auf den Teststreifen und dem Erscheinen des Messwertes. In der US 4,787,398 A wird noch eine relativ lange Messzeit von 60 s beschrieben.

Zur Verkürzung der Messzeit werden unter anderem hydrophil ausgerüstete Vliese oder Gewebe wie in US 6,555,061 B verwendet, um das Blut schneller zum Messbereich (Enzym) zu transportieren. Das Messverfahren ist identisch mit dem in EP 0 451 981 A1 beschriebenen. Bei dem Aufbau des Diagnosestreifens wird ein doppelseitiges Klebeband Scotch ® 415 verwendet.

Ein weiteres Beispiel für einen Streifen ist in US 2002/0102739 A beschrieben. Hier wird Bluttransport von 1,0 mm/s durch ein Plasma behandeltes Gewebe (Docht) erreicht.

Weitere Offenbarungen von Diagnoseteststreifen sind in US D 450,854 S, US 5,304,468 A und US 5,709,837 A zu finden. In den letzten beiden Zitaten wird für den Aufbau des Teststreifens ein so genannter Schmelzklebstoff, der durch Wärmezufuhr klebrig wird, verwendet.

In WO 03/008933 A1 wird ein Diagnoseteststreifen beschrieben, der aus einer Membrane aufgebaut ist, die das Blut chromatographiert, so dass einzelne Blutbestandteile separat untersucht werden können. Im Beispiel 1 ist die Verwendung eines nicht näher beschrieben doppelseitigen Klebebandes mit PET-Träger erwähnt.

Eine Weiterentwicklung zu der kolorimetrischen Messmethode ist die elektrische Bestimmung der Änderung des Oxidations-Potentials an einer mit dem Enzym belegten Elektrode. Dieses Verfahren und ein entsprechender Diagnoseteststreifen sind in WO 01/67099 A1 beschrieben. Der Aufbau des Diagnosestreifens erfolgt durch eine Bedruckung von verschiedenen Funktionsschichten wie elektrischen Leitern, Enzym, und Schmelzklebstoff auf das Basismaterial aus zum Beispiel Polyester. Anschließend wird durch thermische Aktivierung des Klebers ein hydrophiler Film dazukaschiert. Der hydrophile Film dient zum Transport des Bluts zur Messzelle.
Bei diesem Aufbau ist kein Gewebe oder Vlies zum Bluttransport notwendig. Der Vorteil dieses Aufbaus und der Vorteil der neuen Messmethode sind, dass die Messung des Blutzuckergehaltes mit sehr viel weniger Blutvolumen von etwa 5 bis 10 µl und in kürzerer Messzeit stattfinden kann.

In WO 03/067252 A1 wird ebenfalls ein Diagnosetest zur Bestimmung des Blutzuckergehaltes auf Basis der Messung des elektrischen Potentials an einer mit Glucose-Oxidase beschichteten Elektrode offenbart. Der Aufbau des Diagnoseteststreifens ist sehr ähnlich dem in WO 01/67099 A1 beschriebenen. Auch hier wird vorzugsweise ein Schmelzklebstoff mit einem Masseauftrag von 10 bis 50 g/m², bevorzugt 20 bis 30 g/m² verwendet. Alternativ zur Bildung des Streifens ist ebenfalls die Verwendung eines Haftklebebandes möglich.

Die Herstellung der beschriebenen Diagnosestreifen geschieht in den meisten Fällen durch eine diskontinuierliche Abfolge von Beschichtungs- und Laminierschritten. Als Basismaterial dient eine 300 bis 500 µm dicke Folie aus Polyvinylchlorid, Polyester oder Polycarbonat mit den Abmaßen von etwa 400 x 400 mm. Nach den Beschichtungs- und Laminierschritten wird diese Folie zunächst in schmalere Reihen mit Abmaßen von etwa 400 X 40 mm geschnitten. In einem weiteren Schneidvorgang werden letztlich diese Reihen zu den Diagnoseteststreifen geschnitten. Aus einer Reihe entstehen etwa 70 Diagnosestreifen.

Das Schneiden der schmalen Reihen zu den Diagnosestreifen geschieht mit sehr hohen Taktraten von 100 Reihen je Sekunde mit Schneidmaschinen von zum Beispiel der Siebler GmbH oder der Kinematik Inc. Bei diesem Schneidvorgang treten bei den Produkten, die mit einem handelsüblichen Haftklebeband aufgebaut sind, bereits nach kurzer Zeit Verunreinigungen des Schneidwerkzeuges durch Klebemassereste auf. Diese Verunreinigungen sind bereits nach wenigen Stunden so stark, dass die Messer, Antriebseinheiten und Führungsschienen der Schneidmaschine komplett ausgetauscht und gereinigt werden müssen. Dadurch entstehen erhebliche Kosten durch Wartung und Stillstand. Die Schneidwerkzeuge müssen etwa aller 4000 bis 8000 Schnitte gereinigt werden.
Die erwähnten Klebemassereste sind auf die verwendeten handelsüblichen Selbstklebebänder zurückzuführen. Durch die Verwendung von nicht selbstklebrigen Schmelzklebstoffen oder Heißsiegelklebstoffen wie zum Beispiel auf Basis von Polyamiden, Polyisobutylen, Polyvinylbutyral, Polyester, Poly(ethersulfon)en, Ethylen/Ethylacrylat-Copolymeren oder Ethylen/Vinylacetat-Copolymeren wird eine deutliche Verlängerung der Reinigungsinterwalle erreicht. Hier müssen die Schneidwerkzeuge nur aller 8000 Schnitte gereinigt werden.
Bei der Verwendung von Schmelzklebstoffen werden allerdings erhebliche Nachteile im Aufbau der Diagnoseteststreifen beobachtet. Für die Aktivierung der Schmelzklebstoffe sind Druck und Temperaturen von mindestens 80 °C notwendig. Bei diesen Bedingungen besteht zum einen die Gefahr der thermischen Schädigung der Enzymschicht und eines der verwendeten Gewebe oder Vliese, zum anderen ist die Realisierung eines gleichmäßigen Abstandes zwischen den Funktionsschichten wie Basisfolie, Gewebe und Deckfilm des Diagnoseteststreifens nicht möglich. Der Abstand zwischen den Funktionsschichten bestimmt das Blutvolumen, das für die Messung verwendet wird. Bei Schwankungen des Blutvolumens bedingt durch eine zu hohe Schwankungsbreite des Abstandes zwischen den Funktionsschichten bei zum Beispiel unterschiedlichen Teststreifenchargen, ist eine zuverlässige Bestimmung des Blutzuckergehaltes nicht möglich.

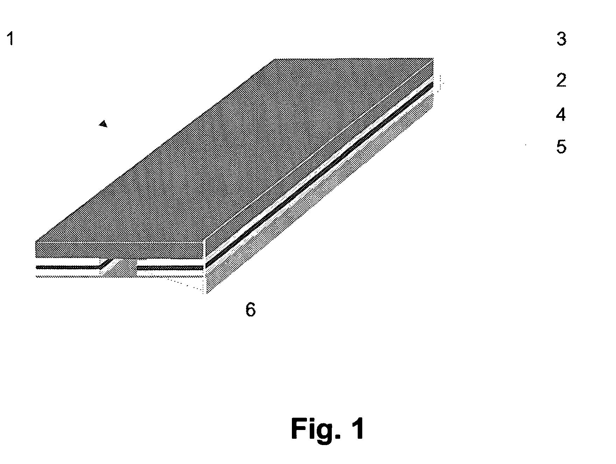

Ein beispielhafter Aufbau eines medizinischen Diagnoseteststreifens ist in Figur 1 schematisch dargestellt.
Der Teststreifen 1 setzt sich aus mehreren einzelnen Schichten 2, 3, 4 und 5 zusammen.
Auf dem Basismaterial 5 aus 500 µm PET befinden sich mehrere vollflächig aufgedruckte Funktionsschichten 4 aus zum Beispiel leitfähigen Materialien oder Enzymen. Diese Funktionsschicht 4 ist mit dem hydrophilen Toptape 3, in diesem Fall einer 100 µm dicken PET-Folie, die einseitig hydrophil ausgerüstet ist, durch zum Beispiel ein Stanzling eines doppelseitigen Haftklebebands 2 verbunden. Das Haftklebeband 2 selbst weist zwei Haftklebeschichten aus vorzugsweise einer Polyacrylat-Haftklebemasse auf, zwischen denen ein Träger aus PET vorhanden ist. Der Stanzling des Haftklebebands 2 bildet einen Kanal 6, der zum Transport der zu vermessenden biologischen Testflüssigkeit zum Beispiel Blut zur Messzelle notwendig ist.

Aufgabe der vorliegenden Erfindung ist, eine bahnförmiges Haftklebeband zur Verfügung zu stellen, das entsprechend der Anforderungen an Diagnoseteststreifen geeignet ist zum Aufbau derselben und im Speziellen im Schneidprozess der Diagnoseteststreifen zu einer erheblich Verringerung der Klebemassereste an den Schneidwerkzeugen führt.

Gelöst wird diese Aufgabe durch ein Haftklebeband, wie es im Hauptanspruch niedergelegt ist. Gegenstand der Unteransprüche sind vorteilhafte Weiterentwicklungen des Erfindungsgegenstandes. Des Weiteren umfasst die Erfindung die Verwendungsmöglichkeit des erfindungsgemäßen Haftklebebandes in medizinischen Diagnosestreifen von biologischen Flüssigkeiten.

Demgemäß betrifft die Erfindung ein Haftklebeband für medizinische Diagnosestreifen, mittels derer biologische Flüssigkeiten untersucht werden, aus einem Trägermaterial, das einseitig oder doppelseitig jeweils mit höchstens 20 g/m², vorzugsweise mit höchstens 15 g/m² einer Haftklebemasse beschichtet ist.
Die Scherfestigkeit der Haftklebemasse bei 25 °C und 70 °C und einer Gewichtsbelastung von 1000 g ist größer als 10 000 min, und das oder die Polymere der Haftklebemasse weisen einen K-Wert von größer als 55 Pa*s auf.

Die charakteristische Eigenschaft des erfindungsgemäßen Haftklebebandes ist die Verwendung einer Haftklebemasse mit hoher Kohäsion beziehungsweise Scherfestigkeit bei gleichzeitig guter Klebkraft mit dünner Klebmasseschicht von höchstens 20 g/m² vorzugsweise von höchstens 15 g/m². Durch diese Kombination von Eigenschaften kann die Aufgabe der Erfindung, die erhebliche Verringerung der Klebemassereste am Schneidwerkzeug im Schneidprozess der Diagnoseteststreifen, gelöst werden. Die hohe Scherfestigkeit der Haftklebemasse äußert sich in einem hohen K-Wert der Polymere beziehungsweise Co-Polymere von größer als 55 Pa*s, bevorzugt von größer als 60 Pa*s, sowie einer hohen Scherfestigkeit von größer 10.000 min bei 70 °C und einer Gewichtsbelastung von 1000 g.

Die hohe Scherfestigkeit der Haftklebemasse spiegelt sich ebenfalls in der Mikroscherwegsuntersuchung wider. Hierbei handelt es sich um eine Methode, bei der innerhalb einer kurzen Messzeit die Scherfestigkeit von Haftklebemassen untersucht werden kann. Der Mikroscherweg µS des Haftklebebandes nach 15 min bei 40 °C unter einer Belastung von 500 g ist vorzugsweise kleiner als 100 µm, weiter vorzugsweise kleiner als 60 µm, besonders bevorzugt kleiner als 30 µm und ganz besonders bevorzugt als 10 µm.

Der Quotient µS2/µS1 als Maß für die Elastizität der Haftklebemasse des erfindungsgemäßen Haftklebebandes ist vorzugsweise kleiner als 0,3 und besonders bevorzugt kleiner als 0,2.

Vorteilhaft ist ebenfalls eine dynamische Glasübergangstemperatur der Polymere beziehungsweise Co-Polymere von -10 °C bis 15 °C und bevorzugt von -6 °C bis 4 °C.

Für den Fachmann überraschend und nicht vorhersehbar kann ein Haftklebeband mit den erfindungsgemäßen Eigenschaften die widersprüchlichen Anforderungen einer guten Klebkraft zu dem Basismaterial der Diagnoseteststreifen bei gleichzeitig geringer Klebrigkeit zu den Schneidwerkzeugen lösen.

Zur Herstellung der Haftklebemasse des erfindungsgemäßen Haftklebebands mit den beschriebenen Eigenschaften eignen sich Copolymere oder Copolymer-Gemische aus Acrylat-Monomeren oder Styrol-Block-Copolymere mit zum Beispiel Ethylen, Propylen, Butylen, Butadien, Hexen und/oder Hexadien als Comonomeren.

Die Haftklebemasse des erfindungsgemäßen Haftklebebands besteht in der bevorzugten Ausführung aus einem oder mehreren Copolymeren aus zumindest den folgenden Monomeren
c1) 79 bis 100 Gew.-% Acrylsäureester und/oder Methacrylsäureester beziehungsweise deren freie Säuren mit der folgenden Formel
   CH₂ = CH(R₁)(COOR₂),
   wobei R₁ = H und/oder CH₃ und R₂ = H und/oder Alkylketten mit 1 bis 30 C-Atomen sind.
   Auch hier können der zugrunde liegenden Monomermischung als weitere Komponente
c2) bis zu 30 Gew.-% olefinisch ungesättigte Monomere mit funktionellen Gruppen zugesetzt sein.

In einer sehr bevorzugten Auslegung werden für die Monomere c1) Acrylmomonere eingesetzt, die Acryl- und Methacrylsäureester mit Alkylgruppen bestehend aus 4 bis 14 C-Atomen, bevorzugt 4 bis 9 C-Atomen umfassen. Spezifische Beispiele, ohne sich durch diese Aufzählung einschränken zu wollen, sind n-Butylacrylat, n-Pentylacrylat, n-Hexylacrylat, n-Heptylacrylat, n-Octylacrylat, n-Nonylacrylat, Laurylacrylat, Stearylacrylat, Behenylacrylat, und deren verzweigten Isomere wie zum Beispiel t-Butylacrylat und 2-Ethylhexylacrylat.

Weitere Verbindungsklassen, die ebenfalls in geringen Mengen unter c1) hinzugesetzt werden können, sind Methylmethacrylate, Cyclohexylmethacrylate, Isobornylacrylat und Isobornylmethacrylate.

In einer sehr bevorzugten Auslegung werden für die Monomere c2) Vinylester, Vinylether, Vinylhalogenide, Vinylidenhalogenide, Vinylverbindungen mit aromatischen Cyclen und Heterocyclen in α-Stellung eingesetzt.
Auch hier seien einige Beispiele genannt, ohne dass die Aufzählung als abschließend zu betrachten ist:
Vinylacetat, Vinylformamid, Vinylpyridin, Ethylvinylether, Vinylchlorid, Vinylidenchlorid und Acrylonitril.
In einer weiteren sehr bevorzugten Auslegung für die Monomere c2) werden Monomere mit folgenden funktionellen Gruppen eingesetzt:
Hydroxy-, Carboxy-, Epoxy-, Säureamid-, Isocyanato- oder Aminogruppen.

In einer vorteilhaften Variante werden für c2) Acrylmonomere entsprechend der allgemeinen Formel
CH₂ = CH(R₁)(COOR₃),
wobei R₁ = H oder CH₃ ist und der Rest R₃ eine funktionelle Gruppe darstellt oder beinhaltet, welche eine nachfolgende UV-Vernetzung der Haftklebemasse unterstützt, welche zum Beispiel in einer besonders bevorzugten Auslegung eine H-Donor Wirkung besitzt.

Besonders bevorzugte Beispiele für die Komponente c2) sind Hydroxyethylacrylat, Hydroxypropylacrylat, Hydroxyethylmethacrylat, Hydroxypropylmethacrylat, Allylalkohol, Maleinsäureanhydrid, Itaconsäureanhydrid, Itaconsäure, Acrylamid und Glyceridylmethacrylat, Benzylacrylat, Benzylmethacrylat, Phenylacrylat, Phenylmethacrylat, t-Butylphenylacrylat, t-Butylaphenylmethacrylat, Phenoxyethylacrlylat, Phenoxyethylmethacrylat, 2-Butoxyethylmethacrylat, 2-Butoxyethylacrylat, Dimethylaminoethylmethacrylat, Dimethylaminoethylacrylat, Diethylaminoethylmethacrylat, Diethylaminoethylacrylat, Cyanoethylmethacrylat, Cyanoethylacrylat, Gycerylmethacrylat, 6-Hydroxyhexylmethacrylat, N-tert.-Butylacrylamid, N-Methylolmethacrylamid, N-(Buthoxymethyl)methacrylamid, N-Methylolacrylamid, N-(Ethoxymethyl)acrylamid, N-Isopropylacrylamid, Vinylessigsäure, Tetrahydrofufurylacrlyat, β-Acryloyloxypropionsäure, Trichloracrylsäure, Fumarsäure, Crotonsäure, Aconitsäure, Dimethylacrylsäure, wobei diese Aufzählung nicht abschließend zu verstehen ist.

In einer weiteren bevorzugten Auslegung werden für die Komponente c2) aromatische Vinylverbindungen eingesetzt, wobei bevorzugt die aromatischen Kerne aus C₄ bis C₁₈ bestehen und auch Heteroatome enthalten können. Besonders bevorzugte Beispiele sind Styrol, 4-Vinylpyridin, N-Vinylphthalimid, Methylstyrol, 3,4-Dimethoxystyrol, 4-Vinylbenzoesäure, wobei diese Aufzählung nicht abschließend zu verstehen ist.

Zur Polymerisation werden die Monomere wiederum dermaßen gewählt, dass die resultierenden Polymere als industriell verwendbare Haftklebemassen eingesetzt werden können, insbesondere derart, dass die resultierenden Polymere haftklebende Eigenschaften entsprechend des "Handbook of Pressure Sensitive Adhesive Technology" von Donatas Satas (van Nostrand, New York 1989) besitzen. Für die Haftklebemasse liegt die statische Glasübergangstemperatur des resultierenden Polymers vorteilhaft zwischen -10 und 15 °C und besonders bevorzugt zwischen -6 und 4 °C.

Zur Herstellung der Polyacrylathaftklebemassen werden vorteilhaft konventionelle radikalische Polymerisationen oder kontrollierte radikalische Polymerisationen durchgeführt. Für die radikalisch verlaufenden Polymerisationen werden bevorzugt Initiatorsysteme eingesetzt, die zusätzlich weitere radikalische Initiatoren zur Polymerisation enthalten, insbesondere thermisch zerfallende radikalbildende Azo- oder Peroxo-Initiatoren. Prinzipiell eignen sich jedoch alle für Acrylate dem Fachmann geläufigen, üblichen Initiatoren. Die Produktion von C-zentrierten Radikalen ist im Houben Weyl, Methoden der Organischen Chemie, Vol. E 19a, Seite 60 bis 147 beschrieben. Diese Methoden werden in bevorzugter Weise in Analogie angewendet.
Beispiele für Radikalquellen sind Peroxide, Hydroperoxide und Azoverbindungen, als Beispiele für typische Radikalinitiatoren seien genannt Kaliumperoxodisulfat, Dibenzoylperoxid, Cumolhydroperoxid, Cyclohexanonperoxid, Di-t-butylperoxid, Azodiisosäurebutyronitril, Cyclohexylsulfonylacetylperoxid, Diisopropyl-percarbonat, t-Butylperoktoat, Benzpinacol.

Die mittleren Molekulargewichte Mₙ der bei der radikalischen Polymerisation entstehenden Haftklebemassen werden sehr bevorzugt derart gewählt, dass sie in einem Bereich von 20.000 bis 2.000.000 g/mol liegen; speziell für die weitere Verwendung als Schmelzhaftkleber werden Haftklebemassen mit mittleren Molekulargewichten Mₙ von 100.000 bis 500.000 g/mol hergestellt. Die Bestimmung des mittleren Molekulargewichtes erfolgt über Größenausschlusschromatographie (SEC) oder Matrix-unterstützte Laser-Desorption/lonisations-Massenspektrometrie (MALDI-MS).

Die Polymerisation kann in Substanz, in Gegenwart eines oder mehrerer organischer Lösungsmittel, in Gegenwart von Wasser oder in Gemischen aus organischen Lösungsmitteln und Wasser durchgeführt werden. Es wird dabei angestrebt, die verwendete Lösungsmittelmenge so gering wie möglich zu halten. Geeignete organische Lösungsmittel sind reine Alkane (zum Beispiel Hexan, Heptan, Octan, Isooctan), aromatische Kohlenwasserstoffe (zum Beispiel Benzol, Toluol, Xylol), Ester (zum Beispiel Essigsäureethylester, Essigsäurepropyl-, -butyl- oder -hexylester), halogenierte Kohlenwasserstoffe (zum Beispiel Chlorbenzol), Alkanole (zum Beispiel Methanol, Ethanol, Ethylenglycol, Ethylenglycolmonomethylether) und Ether (zum Beispiel Diethylether, Dibutylether) oder Gemische davon.
Die wässrigen Polymerisationsreaktionen können mit einem mit Wasser mischbaren oder hydrophilen Colösungsmittel versetzt werden, um zu gewährleisten, dass das Reaktionsgemisch während des Monomerumsatzes in Form einer homogenen Phase vorliegt. Vorteilhaft verwendbare Colösungsmittel für die vorliegende Erfindung werden gewählt aus der folgenden Gruppe, bestehend aus aliphatischen Alkoholen, Glycolen, Ethern, Glycolethern, Pyrrolidinen, N-Alkylpyrrolidinonen, N-Alkylpyrrolidonen, Polyethylenglycolen, Polypropylenglycolen, Amiden, Carbonsäuren und Salzen davon, Estern, Organosulfiden, Sulfoxiden, Sulfonen, Alkoholderivaten, Hydroxyetherderivaten, Aminoalkoholen, Ketonen und dergleichen sowie Derivaten und Gemischen davon.

Zur Radikalstabilisierung werden in günstiger Vorgehensweise Nitroxide des Typs (NIT 1) oder (NIT 2) eingesetzt: 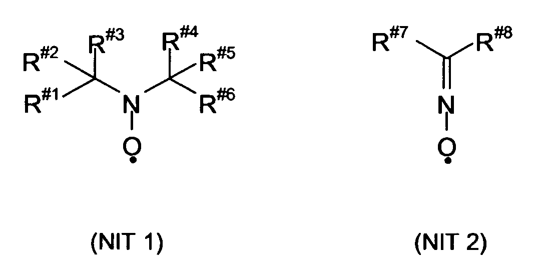 wobei R^{#1}, R^{#2}, R^{#3}, R^{#4}, R^{#5}, R^{#6}, R^{#7}, R^{#8} unabhängig voneinander folgende Verbindungen oder Atome bedeuten:
i) Halogenide, wie zum Beispiel Chlor, Brom oder lod
ii) lineare, verzweigte, cyclische und heterocyclische Kohlenwasserstoffe mit 1 bis 20 Kohlenstoffatomen, die gesättigt, ungesättigt oder aromatisch sein können,
iii) Ester -COOR^{#9}, Alkoxide -OR^{#10} und/oder Phosphonate -PO(OR^{#11})₂,
   wobei R^{#9}, R^{#10} und/oder R^{#11} für Reste aus der Gruppe ii) stehen.

Verbindungen der Struktur (NIT 1) oder (NIT 2) können auch an Polymerketten jeglicher Art gebunden sein (vorrangig in dem Sinne, dass zumindest einer der oben genannten Reste eine derartige Polymerkette darstellt) und somit zum Aufbau der Blockcopolymere als Makroradikale oder Makroregler genutzt werden.

Eine Reihe weiterer Polymerisationsmethoden, nach denen die Polyacrylathaftklebemasse in alternativer Vorgehensweise hergestellt werden können, sind aus dem Stand der Technik bekannt.

Die US 4,581,429 A offenbart ein kontrolliert radikalisches Polymerisationsverfahren, das als Initiator eine Verbindung der Formel R'R"N-O-Y anwendet, worin Y eine freie radikalische Spezies ist, die ungesättigte Monomere polymerisieren kann. Die Reaktionen weisen aber im Allgemeinen geringe Umsätze auf. Besonders problematisch ist die Polymerisation von Acrylaten, die nur zu sehr geringen Ausbeuten und Molmassen abläuft.

WO 98/13392 A1 beschreibt offenkettige Alkoxyaminverbindungen, die ein symmetrisches Substitutionsmuster aufweisen.

Die EP 0 735 052 A1 offenbart ein Verfahren zur Herstellung thermoplastischer Elastomere mit engen Molmassenverteilungen.

WO 96/24620 A1 beschreibt ein Polymerisationsverfahren, bei dem sehr spezielle Radikalverbindungen wie zum Beispiel phosphorhaltige Nitroxide, die auf Imidazolidin basieren, eingesetzt werden.

WO 98/44008 A1 offenbart spezielle Nitroxyle, die auf Morpholinen, Piperazinonen und Piperazindionen basieren.

DE 199 49 352 A1 beschreibt heterozyklische Alkoxyamine als Regulatoren in kontrolliert radikalischen Polymerisationen. Entsprechende Weiterentwicklungen der Alkoxyamine beziehungsweise der korrespondierenden freien Nitroxide verbessern die Effizienz zur Herstellung von Polyacrylaten (Hawker, Beitrag zur Hauptversammlung der American Chemical Society, Frühjahr 1997; Husemann, Beitrag zum IUPAC World-Polymer Meeting 1998, Gold Coast).

Als weitere kontrollierte Polymerisationsmethode lässt sich in vorteilhafter Weise zur Synthese der Blockcopolymere die Atom Transfer Radical Polymerization (ATRP) einsetzen, wobei als Initiator bevorzugt monofunktionelle oder difunktionelle sekundäre oder tertiäre Halogenide und zur Abstraktion des(r) Halogenids(e) Cu-, Ni-, Fe-, Pd-, Pt-, Ru-, Os-, Rh-, Co-, Ir-, Ag- oder Au-Komplexe (gemäß der EP 0 824 111 A1, der EP 0 826 698 A1, der EP 824 110 A1, der EP 841 346 A1 oder der EP 850 957 A1) eingesetzt werden. Die unterschiedlichen Möglichkeiten der ATRP sind ferner in der US 5,945,491 A, der US 5,854,364 A und der US 5,789,487 A beschrieben.

Weiterhin vorteilhaft kann das erfindungsgemäß genutzte Polymer über eine anionische Polymerisation hergestellt werden. Hier werden als Reaktionsmedium bevorzugt inerte Lösungsmittel verwendet wie zum Beispiel aliphatische und cycloaliphatische Kohlenwasserstoffe oder auch aromatische Kohlenwasserstoffe.

Weiterhin lassen sich auch difunktionelle Initiatoren einsetzen wie beispielsweise 1,1,4,4-Tetraphenyl-1,4-dilithiobutan oder 1,1,4,4-Tetraphenyl-1,4-dilithioisobutan. Coinitiatoren lassen sich ebenfalls einsetzen. Geeignete Coinitiatoren sind unter anderem Lithiumhalogenide, Alkalimetallalkoxide oder Alkylaluminium-Verbindungen. In einer sehr bevorzugten Version sind die Liganden und Coinitiatoren so gewählt, dass Acrylatmonomere wie zum Beispiel n-Butylacrylat und 2-Ethylhexylacrylat direkt polymerisiert werden können und nicht im Polymer durch eine Umesterung mit dem entsprechenden Alkohol generiert werden müssen.

Als sehr bevorzugter Herstellprozess wird eine Variante der RAFT-Polymerisation (reversible addition-fragmentation chain transfer polymerization) durchgeführt. Der Polymerisationsprozess ist zum Beispiel in der WO 98/01478 A1 und der WO 99/31144 A1 ausführlich gezeigt. Zur Herstellung eignen sich besonders vorteilhaft Trithiocarbonate der allgemeinen Struktur R'''-S-C(S)-S-R''' (Macromolecules 2000, 33, 243 bis 245).

In Verbindung mit den oben genannten kontrolliert radikalisch verlaufenden Polymerisationen werden Initiatorsysteme bevorzugt, die zusätzlich weitere radikalische Initiatoren zur Polymerisation enthalten, insbesondere thermisch zerfallende radikalbildende Azo- oder Peroxoinitiatoren. Prinzipiell eignen sich hierfür jedoch alle für Acrylate bekannten üblichen Initiatoren. Die Produktion von C-zentrierten Radikalen ist im Houben-Weyl, Methoden der Organischen Chemie, Vol. E19a, S. 60ff beschrieben. Diese Methoden werden in bevorzugter Weise angewendet.
Beispiele für Radikalquellen sind Peroxide, Hydroperoxide und Azoverbindungen. Als einige nicht ausschließliche Beispiele für typische Radikalinitiatoren seinen hier genannt Kaliumperoxodisulfat, Dibenzoylperoxid, Cumolhydroperoxid, Cyclohexanonperoxid, Cyclohexylsulfonylacetylperoxid, Di-tert-butylperoxid, Azodiisobutyronitril, Diisopropylpercarbonat, tert-Butylperoctoat, Benzpinacol. In einer sehr bevorzugten Variante wird als radikalischer Initiator 1,1'-Azo-bis-(cyclohexylnitril) (Vazo 88®, DuPont®) oder 2,2-Azo-bis-(2-methylbutannitril) (Vazo 67®, DuPont®) verwendet. Weiterhin können auch Radikalquellen verwendet werden, die erst unter UV-Bestrahlung Radikale freisetzen.

Beim konventionellen RAFT-Prozess wird zumeist nur bis zu geringen Umsätzen polymerisiert (WO 98/01478 A1), um möglichst enge Molekulargewichtsverteilungen zu realisieren. Durch die geringen Umsätze lassen sich diese Polymere aber nicht als Haftklebemassen und insbesondere nicht als Schmelzhaftkleber einsetzen, da der hohe Anteil an Restmonomeren die klebtechnischen Eigenschaften negativ beeinflusst, die Restmonomere im Aufkonzentrationsprozess das Lösemittelrecyclat verunreinigen und die entsprechenden Selbstklebebänder ein sehr hohes Ausgasungsverhalten zeigen würden.

Bevorzugt wird die innere Festigkeit (Kohäsion) der Polyacrylhaftklebemasse des erfindungsgemäßen Haftklebebandes durch eine Vernetzung gesteigert. Durch die Vernetzung der Haftklebemasse erhöhen sich der Gelwert und der Mikroscherweg des erfindungsgemäßen Haftklebebandes. Allerdings verringert sich durch die Vernetzung auch die Klebkraft. Für die Vernetzung können den acrylathaltigen Haftklebemassen optional verträgliche Vernetzersubstanzen zugesetzt werden. Als Vernetzer eignen sich besonders Metallchelate, multifunktionelle Isocyanate, multifunktionelle Amine oder multifunktionelle Alkohole. Die Vernetzung kann in günstiger Weise thermisch oder durch energiereiche Strahlung (actinische Strahlung) erfolgen, im letzteren Fall insbesondere durch Elektronenstrahlung (ES) oder nach Zugabe geeigneter Photoinitiatoren durch ultraviolette Strahlung. Bevorzugte unter Strahlung vernetzende Substanzen sind zum Beispiel bi- oder multifunktionelle Acrylate oder bi- oder multifunktionelle Urethanacrylate, bi- oder multifunktionelle Isocyanate oder bi- oder multifunktionelle Epoxide. Verwendet werden können hier aber auch alle weiteren, dem Fachmann geläufigen bi- oder multifunktionellen Verbindungen, die in der Lage sind, Polyacrylate zu vernetzen. Als Photoinitiatoren eignen sich bevorzugt Norrish-Typ I - und -Typ II -Spalter, wobei einige Beispiele für beide Klassen sein können Benzophenon-, Acetophenon-, Benzil-, Benzoin-, Hydroxyalkylphenon-, Phenylcyclohexylketon-, Anthrachinon-, Thioxanthon-, Triazin-, oder Fluorenonderivate, wobei diese Aufzählung keinen Anspruch auf Vollständigkeit besitzt, sondern ohne erfinderisches Zutun vom Fachmann erweitert werden kann.

Zur vorteilhaften Weiterentwicklung sind der Polyacrylathaftklebemassen des erfindungsgemäßen Haftklebebandes keinerlei Additive wie klebrigmachende Harze oder Weichmacher (Plastifizierungsmittel) zugesetzt. Derartige Additive erhöhen zwar die Klebkraft, verringern aber erheblich die Scherfestigkeit der Haftklebemasse und führen somit zu Klebmasseresten an den Schneidwerkzeugen beim Schneidprozess der Diagnoseteststreifen.

Additive wie Füllstoffe (zum Beispiel Fasern, Ruß, Zinkoxid, Titandioxid, Kreide, Voll- oder Hohlglaskugeln, Mikrokugeln aus anderen Materialien, Kieselsäure, Silikate, Nanopartikel), Compoundierungsmittel und/oder Alterungsschutzmittel, zum Beispiel in Form von primären und sekundären Antioxidantien oder in Form von Lichtschutzmitteln können der Haftklebemasse zugesetzt werden.

Zusammenfassend weist die bevorzugte Ausführungsform des erfindungsgemäßen Haftklebebands eine Polyacrylathaftklebemasse auf, die durch Coextrusion, Schmelz-, Lösemittel- oder Dispersionsbeschichtung gefertigt wird. Besonders bevorzugt ist eine Kommarakelbeschichtung der Polyacrylathaftklebemasse aus einem geeigneten Lösemittel oder Lösemittelgemisch.

Vorteilhaft ist die Verwendung einer Primerschicht zwischen Trägerfolie und Polyacrylathaftklebemasse zur Verbesserung der Haftung der Klebmasse auf der Trägerfolie und somit zur Vermeidung von Klebmasseresten auf dem Schneidwerkzeug beim Schneidprozess der Diagnoseteststreifen. Als Primer sind die bekannten Dispersion- und Lösungsmittelsysteme verwendbar zum Beispiel auf Basis von Isopren- oder Butadienhaltigen Kautschuk, Cyclokautschuk, Polyvinylchlorid- und/oder Polyvinyldichlorid Homo- oder Copolymeren. Isocyanate oder Epoxyharze als Additive verbessern die Haftung und erhöhen zum Teil auch die Scherfestigkeit des Haftklebstoffes. Physikalische Oberflächenbehandlungen wie Beflammung, Corona oder Plasma oder Coextrusionsschichten sind ebenfalls geeignet, die Haftung zu verbessern.

Im Falle der einseitigen Beschichtung der Trägerfolie mit der Polyacrylathaftklebemasse kann die Rückseite der Trägerfolie mit einem der bekannten Releasemittel (gegebenenfalls mit anderen Polymeren abgemischt) beschichtet sein. Beispiele sind Stearyl-Verbindungen (zum Beispiel Polyvinylstearylcarbamat, Stearylverbindungen von Übergangsmetallen wie Cr oder Zr, Harnstoffe aus Polyethylenimin und Stearylisocyanat, Polysiloxane (zum Beispiel als Copolymer mit Polyurethanen oder als Propfcopolymer auf Polyolefin), thermoplastische Fluorpolymere. Der Begriff Stearyl steht als Synonym für alle geraden oder verzweigten Alkyle oder Alkenyle mit einer C-Zahl von mindestens 10, wie zum Beispiel Octadecyl.

Beschreibungen der üblichen Klebmassen sowie Rückseitenbeschichtungen und Primern finden sich zum Beispiel in "Handbook of Pressure Sensitive Adhesive Technology", D. Satas, (3. Auflage).

Als Trägermaterialien für das Haftklebeband werden die dem Fachmann geläufigen und üblichen Trägermaterialien wie Folien aus Polyester, Polyethylen, Polypropylen, Verstreckten Polypropylen, Polyvinylchlorid, besonders bevorzugt Folien aus Polyethylenterephthalat (PET) verwendet. Diese Aufzählung ist nicht abschließend zu verstehen, sondern im Rahmen der Erfindung sind weitere Folien enthalten.
Das Trägermaterial kann vorzugsweise ein- oder beidseitig mit der Polyacrylathaftklebemasse ausgerüstet sein. Die Trägerfolie kann ein- oder zweiseitig unter Verwendung der üblichen Druckverfahren bedruckt sein. Das Selbstklebeband kann ebenfalls mit einer handelsüblichen Trennfolie, die üblicherweise aus einem Basismaterial aus Polyethylen, Polypropylen, Polyester oder Papier das ein- oder doppelseitig mit Polysiloxan beschichtet ist, zusammenkaschiert sein.

Für die Verarbeitung und Verwendung im Diagnoseteststreifen kann es von Vorteil sein, wenn aus dem erfindungsgemäßen Haftklebeband Stanzlinge mit einer für die Anwendung geeigneten Stanzform hergestellt werden.

Die Gesamtdicke des Haftklebebands beträgt vorzugsweise ohne Trennfolie 20 bis 150 µm, vorzugsweise 50 bis 100 µm.
Weiter vorzugsweise beträgt die Klebkraft auf Stahl mindestens 1,5 und vorzugsweise 2,5 N/cm und/oder die Klebkraft auf PET mindestens 0,5 und vorzugsweise 1,0 N/cm.

Für die erfindungsgemäß bevorzugte Verwendung des Haftklebebandes in einem Diagnoseteststreifen sind neben der biologischen Kompatibilität der Bestandteile zur biologischen Testflüssigkeit und zur Enzymreaktion auch die Dickentoleranz und eine geringe Kompressibilität wichtig. Da durch die Dicke des Haftklebebandes bei den meisten Diagnoseteststreifen der Abstand zwischen den Funktionsschichten wie Basisfolie, Gewebe und Deckfilm und damit das Volumen der biologischen Testflüssigkeit im Teststreifen bestimmt wird, ist nur durch eine geringe Kompressibilität sowie durch eine sehr gute Dickentoleranz eine korrekte Messung zum Beispiel des Blutzuckergehaltes möglich.

### Prüfmethoden

### K-Wert

Der K-Wert ist ein Maß für die mittlere Molekülgröße hochpolymerer Stoffe. Das Prinzip der Methode beruht auf der kapillarviskosimetrischen Bestimmung der relativen Lösungsviskosität. Hierzu wird die Testsubstanz in Toluol durch dreißigminütiges Schütteln aufgelöst, so dass man eine 1 %-ige Lösung erhält. In einem Vogel-Ossag-Viskosimeter wird bei 25 °C die Auslaufzeit gemessen und daraus in Bezug auf die Viskosität des reinen Lösungsmittels die relative Viskosität der Probenlösung bestimmt. Aus Tabellen kann nach Fikentscher [P. E. Hinkamp, *Polymer,* 1967, 8, 381] der K-Wert abgelesen werden (K = 1000 k).

### Glasübergang

Der dynamische Glasübergang der Haftklebemasse wird mittels rheometrischer Untersuchung bestimmt. Es wird ein Rheometer der Reihe Ares der Firma TA verwendet. Die Glasübergangstemperatur ist das Maximum der tan δ (=G"/G') Kurve und wird bei 10 rad/s ermittelt.

### Gelwert

Die lösungsmittelfreien Haftklebemassenproben werden in ein Vliestütchen aus Polyethylen (Tyvek-Vlies) eingeschweißt. Lösliche Bestandteile werden mit Toluol über eine Dauer von drei Tagen unter täglichem Lösungsmittelaustausch extrahiert. Aus der Differenz der Probengewichte vor der Extraktion und nach der Extraktion wird der Gelwert als prozentuale Angabe des Gewichtsanteils des Polymers, das nicht mit Toluol extrahierbar ist, bestimmt.

### Klebkraft

Die Prüfung der Schälfestigkeit (Klebkraft) erfolgte in Anlehnung an PSTC-1. Ein 2 cm breiter Streifen des Haftklebebandes wird auf dem Prüfuntergrund wie zum Beispiel einer geschliffenen Stahlplatte oder einer PET-Platte durch fünfmaliges doppeltes Überrollen mittels einer 5 kg Rolle verklebt. Die Platte wird eingespannt und der Selbstklebestreifen über sein freies Ende an einer Zugprüfmaschine unter einem Schälwinkel von 180° mit einer Geschwindigkeit von 300 mm/min abgezogen und die dafür notwendige Kraft ermittelt. Die Messergebnisse sind in N/cm angegeben und über drei Messungen gemittelt. Alle Messungen wurden bei Raumtemperatur durchgeführt.

### Scherstandzeiten

Die Prüfung erfolgte in Anlehnung an PSTC-7. Ein 1,3 cm breiter Streifen des Haftklebebandes wird auf einem polierten Stahlplättchen auf einer Länge von 2 cm mit einer 2 kg-Rolle durch zweimaliges doppeltes Überrollen verklebt. Die Plättchen werden für 30 min unter Testbedingungen (Temperatur und Luftfeuchtigkeit), aber ohne Last equilibriert. Dann wird das Testgewicht angehängt, so dass eine Scherbeanspruchung parallel zur Verklebungsfläche entsteht, und die Zeit gemessen, bis die Verklebung versagt. Ist eine Haltezeit von 10 000 min erreicht, so wird der Versuch vor Versagen der Klebbindung abgebrochen.

### Mikroscherweg µS1

Ein 1 cm breiter Streifen des Haftklebebandes wird auf einem polierten Stahlplättchen (Prüfuntergrund) auf einer Länge von 5 cm mit einer 2 kg-Rolle durch dreimaliges doppeltes Überrollen verklebt. Doppelseitige Klebebänder werden auf der Rückseite mit einer 50 µm Aluminiumfolie abgedeckt. Der Teststreifen wird mit einer 190 µm dicken PET-Folie verstärkt und anschließend kantengerade mit Hilfe einer Fixiervorrichtung abgeschnitten. Dabei steht die Kante des verstärkten Teststreifens 1 mm über der Kante des Stahlplättchens. Die Plättchen werden für 15 min unter Testbedingungen (40 °C, 50% rel. Luftfeuchte) im Messgerät, aber ohne Last, equilibriert. Dann wird das Testgewicht 500 g angehängt, so dass eine Scherbeanspruchung parallel zur Verklebungsfläche entsteht. Mittels eines Mikrowegaufnehmers wird in Abhängigkeit der Zeit der Scherweg graphisch aufgenommen.
Als Mikroscherweg µS1 wird die Scherstrecke nach einer Gewichtsbelastung von 15 min angegeben. Nach der Messzeit von 15 min unter Gewichtsbelastung wird das Gewicht vorsichtig von der Probe entfernt und anschließend das Relaxieren für weitere 15 min beobachtet. Nach 15 min ohne Gewichtsbelastung (Relaxtion) wird der Mikroscherweg µS2 ermittelt. Aus den beiden Messwerten wird der Mikroscherwegsquotient µS2/µS1 ermittelt. Dieser Quotient ist ein Maß für die Elastizität der Haftklebemasse.

Im Folgenden soll die Erfindung anhand mehrerer Beispiele näher erläutert werden, ohne damit die Erfindung unnötig einschränken zu wollen.

### Beispiele

### Beispiel 1

Ein für eine radikalische Polymerisation konventioneller Reaktor wurde mit 8 kg Acrylsäure, 45 kg n-Butylacrylat, 3 kg t-Butylacrylat und 60 kg Aceton befüllt. Nach 45 Minuten Durchleiten von Stickstoffgas unter Rühren wurde der Reaktor auf 58 °C hochgeheizt und 20 g Azoisobutyronitril (AIBN, Vazo 6®, Firma DuPont) hinzugegeben. Anschließend wurde das äußere Heizbad auf 75 °C erwärmt, und die Reaktion konstant bei dieser Außentemperatur durchgeführt. Nach 1 h Reaktionszeit wurde wiederum 20 g AIBN hinzugegeben. Nach 3 h und 6 h wurde mit jeweils 10 kg Aceton/Isopropanol (97:3) das Gemisch verdünnt. Zur Reduktion der Restinitiatoren wurden nach 8 h und nach 10 h jeweils 100 g Bis-(4-tert.-Butylcyclohexanyl)-Peroxy-dicarbonat (Perkadox 16®, Firma Akzo Nobel) hinzugegeben. Die Reaktion wurde nach 22 h Reaktionszeit abgebrochen und auf Raumtemperatur abgekühlt.
Nach der Polymerisation wurde das Polymer mit Isopropanol auf einen Feststoffgehalt von 25 % verdünnt und dann mit 0,3 Gew.-% Polyisocyanat (Desmodur N 75, Firma Bayer) unter Rühren abgemischt. Anschließend wurde die Polymerlösung mit Hilfe eine Komma-Rakels auf beiden Seiten eines 50 µm dicken Polyesterträgers, der zuvor je Seite mit 1 g/m² Polyvinyldichchorid-Acrylnitril-Copolymer (Saran, Firma Dow Chemicals) wurde, beschichtet. Die Trocknung erfolgte für 10 Minuten bei 120 °C. Der Masseauftrag je Beschichtungsseite betrug 12 g/m². Nach dem ersten Beschichtungsschritt wurde die Klebmasse mit einem Trennpapier abgedeckt.

### Beispiel 2

Ein für eine radikalische Polymerisation konventioneller Reaktor wurde mit 28 kg Acrylsäure, 292 kg 2-Ethylhexylacrylat, 40 kg Methylacrylat und 300 kg Aceton/Isopropanol (97:3) befüllt. Nach 45 Minuten Durchleiten von Stickstoffgas unter Rühren wurde der Reaktor auf 58 °C hochgeheizt und 0,2 kg Azoisobutyronitril (AIBN, Vazo 64®, Firma DuPont) hinzugegeben. Anschließend wurde das äußere Heizbad auf 75 °C erwärmt, und die Reaktion konstant bei dieser Außentemperatur durchgeführt. Nach 1 h Reaktionszeit wurde wiederum 0,2 kg AIBN hinzugegeben. Nach 3 h und 6 h wurde mit jeweils 150 kg Aceton/Isopropanol (97:3) das Gemisch verdünnt. Zur Reduktion der Restinitiatoren wurden nach 8 h und nach 10 h jeweils 0,4 kg Bis-(4-tert.-Butylcyclohexanyl)-Peroxydicarbonat (Perkadox 16®, Firma Akzo Nobel) hinzugegeben. Die Reaktion wurde nach 22 h Reaktionszeit abgebrochen und auf Raumtemperatur abgekühlt.
Nach der Polymerisation wurde das Polymer mit Isopropanol auf einen Feststoffgehalt von 25 % verdünnt und dann mit 0,4 Gew.-% Aluminium(III)-acetylacetonat unter Rühren abgemischt. Anschließend wurde die Polymerlösung mit Hilfe eine Komma-Rakels auf beiden Seiten eines 50 µm dicken Polyesterträgers, der zuvor mittels Corona vorbehandelt wurde, beschichtet. Die Trocknung erfolgte für 10 Minuten bei 120 °C. Der Masseauftrag je Beschichtungsseite betrug 12 g/m². Nach dem ersten Beschichtungsschritt wurde die Klebmasse mit einem Trennpapier abgedeckt.

### Beispiel 3

Eine Haftklebmassenlösung analog Beispiel 2 wurde mit Hilfe eines Komma-Rakels auf einen bedruckten 50 µm dicken Polyesterträger, der zuvor mittels Corona vorbehandelt und auf der anderen Seite mit einem Releaselack aus Polyvinylstearylcarbamat beschichtet wurde, beschichtet. Die Trocknung erfolgte für 10 Minuten bei 120 °C. Der Masseauftrag je Beschichtungsseite betrug 12 g/m².

| | **Beispiel 1** | **Beispiel 2** | **Beispiel 3** |
|---|---|---|---|
| Haftklebemasse | Rein-Acrylat | Rein-Acrylat | Rein-Acrylat |
| Klebmasseauftrag je Seite [g/m²] | 12 | 12 | 12 |
| Gesamtdicke Haftklebeband o. Trennfolie [µm] | 73 | 74 | 61 |
| K-Wert 25 °C der Haftklebemasse [Pa*s] | 74 | 62 | 62 |
| Glasübergangstemperatur der Haftklebemasse [°C] | 0 | -5 | -5 |
| Mikroschenweg 500g, 40 °C [µm] | 23 | 49 | 51 |
| Quotient µS2/µS1 | 0,15 | 0,19 | 0,18 |
| Scherfestigkeit bei 70 °C [min] | > 10.000 | > 10.000 | > 10.000 |
| Klebkraft auf Stahl [N/cm] | 2,5 | 3,5 | 3,2 |
| Klebkraft auf PET [N/cm] | 1,0 | 1,3 | 1,2 |
| Schneidversuche (8000 schnitte) | Minimale Klebmassereste nach 8000 Schnitten | Geringe Klebmassereste nach 8000 Schnitten | Geringe Klebmassereste nach 8000 Schnitten |

### Gegenbeispiele

### Gegenbeispiel 1

Als Gegenbeispiel 1 wird das kommerzielle Produkt tesa ® 4980 verwendet. Bei diesem Klebeband handelt es sich um ein doppelseitiges Haftklebeband, das aus einem 12 µm PET-Trägermaterial besteht, dass von beiden Seiten mit 34 g/m² einer harzmodifizierten Acrylat-Haftklebemasse beschichtet ist.

### Gegenbeispiel 2

Das Haftklebeband wird wie in Beispiel 1 beschrieben hergestellt. Als Trägerfolie kommt ein 25 µm PET-Film zum Einsatz. Als Haftklebmasse wird die in Beispiel 2 beschriebene mit einem Masseauftrag von 50g/m² je Beschichtungsseite verwendet.

### Gegenbeispiel 3

Als Gegenbeispiel 3 wird das kommerzielle Produkt tesa ® 4972 verwendet. Bei diesem Klebeband handelt es sich um ein doppelseitiges Haftklebeband, das aus einem 12 µm PET-Trägermaterial besteht, dass von beiden Seiten mit 18 g/m² einer harzmodifizierten Acrylat-Haftklebemasse beschichtet ist.

### Gegenbeispiel 4

Als Gegenbeispiel 4 wird das kommerzielle Produkt Scotch ® 415 von 3M verwendet. Bei diesem Klebeband handelt es sich um ein doppelseitiges Haftklebeband, das aus einem 50 µm PET-Trägermaterial besteht, dass von beiden Seiten mit 25 g/m² einer Rein-Acrylat-Haftklebemasse beschichtet ist.

| | **Gegenbeispiel 1** | **Gegenbeispiel 2** | **Gegenbeispiel 3** | **Gegenbeispiel 4** |
|---|---|---|---|---|
| Haftklebemasse | Acrylat Harzmodifiziert | Rein-Acrylat | Acrylat Harzmodifiziert | Rein-Acrylat |
| Klebmasseauftrag je Seite [g/m²] | 34 | 50 | 18 | 25 |
| Gesamtdicke Haftklebeband o. Trennfolie [µm] | 80 | 125 | 48 | 100 |
| K-Wert 25 °C der Haftklebemasse [Pa*s] | 57 | 62 | 57 | nicht bekannt |
| Glasübergangstemperatur der Haftklebemasse [°C] | 5 | -5 | 5 | -4 |
| Mikroscherweg µS1 500g, 40 °C [µm] | 470 | 52 | 195 | 550 |
| Quotient µS2/µS1 | 0,31 | 0,19 | 0,32 | 0,35 |
| Scherfestigkeit bei 70 °C [min] | 1278 | > 10.000 | 1322 | 1269 |
| Klebkraft auf Stahl [N/cm] | 8,3 | 5,3 | 7,0 | 2,7 |
| Klebkraft auf PET [N/cm] | 6,5 | 4,2 | 5,3 | 2,2 |
| Schneidversuche (8000 Schnitte) | Starke Klebmassereste Abbruch nach 2000 Schnitten | Klebmassereste Abbruch nach 4500 Schnitten | Starke Klebmassereste Abbruch nach 3000 Schnitten | Starke Klebmassereste Abbruch nach 2000 Schnitten |

## Patentansprüche

1. Haftklebeband für medizinische Diagnosestreifen, mittels derer biologische Flüssigkeiten untersucht werden, aus einem Trägermaterial, das einseitig oder doppelseitig jeweils mit höchstens 20 g/m², vorzugsweise mit höchstens 15 g/m² einer Haftklebemasse beschichtet ist, wobei die Scherfestigkeit der Haftklebemasse bei 25 °C und 70 °C und einer Gewichtsbelastung von 1000 g größer als 10 000 min ist und das oder die Polymere der Haftklebemasse einen K-Wert von größer als 55 Pa*s aufweisen.

2. Haftklebeband nach Anspruch 1, **dadurch gekennzeichnet, dass** die Haftklebemasse aus einem oder mehreren Copolymeren, in denen Acrylat-Monomeren den Hauptbestandteil bilden, besteht.

3. Haftklebeband nach den Ansprüchen 1 oder 2, **dadurch gekennzeichnet, dass** die Polymere der Haftklebemasse einen K-Wert von größer als 60 Pa*s haben.

4. Haftklebeband nach zumindest einem der Ansprüche 1 bis 3, **dadurch gekennzeichnet, dass** der Mikroscherweg des Haftklebebandes nach 15 min bei 40 °C unter einer Belastung von 500 g kleiner als 100 µm, vorzugsweise kleiner als 60 µm und besonders bevorzugt kleiner als 30 µm ist.

5. Haftklebeband nach zumindest einem der vorherigen Ansprüche, **dadurch gekennzeichnet, dass** der Quotient der Mikroscherwege µS2/µS1 kleiner als 0,3, vorzugsweise kleiner als 0,2 ist.

6. Haftklebeband nach zumindest einem der vorherigen Ansprüche, **dadurch gekennzeichnet, dass** die dynamische Glasübergangstemperatur der Haftklebemasse bei 10 rad/s -10 °C bis 15 °C und bevorzugt -6 °C bis 4 °C beträgt.

7. Haftklebeband nach zumindest einem der vorherigen Ansprüche, **dadurch gekennzeichnet, dass** die Haftklebemassen keinerlei Zusätze an Klebharzen oder Weichmachern enthält.

8. Haftklebeband nach zumindest einem der vorherigen Ansprüche, **dadurch gekennzeichnet, dass** die Gesamtdicke des Haftklebebands ohne Trennfolie 20 bis 150 µm, vorzugsweise 50 bis 100 µm beträgt.

9. Haftklebeband nach zumindest einem der vorherigen Ansprüche, **dadurch gekennzeichnet, dass** die Klebkraft auf Stahl mindestens 1,5 und vorzugsweise 2,5 N/cm und/oder die Klebkraft auf PET mindestens 0,5 und vorzugsweise 1,0 N/cm beträgt.

10. Haftklebeband nach zumindest einem der vorherigen Ansprüche, **dadurch gekennzeichnet, dass** die Trägerfolie aus PET besteht.

11. Haftklebeband nach zumindest einem der vorherigen Ansprüche, **dadurch gekennzeichnet, dass** sich zwischen dem Trägermaterial und der Haftklebemassen ein Haftvermittler befindet.

12. Verwendung eines Haftklebebands nach zumindest einem der vorherigen Ansprüche in medizinischen Diagnosestreifen, mittels derer biologische Flüssigkeiten untersucht werden.

## Claims

1. Pressure-sensitive adhesive tape for medical diagnostic strips used to analyze biological fluids, the tape comprising a carrier material coated on one or both sides with not more than 20 g/m², preferably not more than 15 g/m², per side, of a pressure-sensitive adhesive, where the shear strength of the pressure-sensitive adhesive at 25 °C and 70 °C under a weight load of 1000 g is greater than 10 000 min and the polymer or polymers of the pressure-sensitive adhesive has or have a K value of greater than 55 Pa*s.

2. Pressure-sensitive adhesive tape according to Claim 1, **characterized in that** the pressure-sensitive adhesive is composed of one or more copolymers in which acrylate monomers form the principal constituent.

3. Pressure-sensitive adhesive tape according to Claims 1 or 2, **characterized in that** the polymers of the pressure-sensitive adhesive have a K value of greater than 60 Pa*s.

4. Pressure-sensitive adhesive tape according at least one of Claims 1 to 3, **characterized in that** the microshear travel of the pressure-sensitive adhesive tape after 15 minutes at 40 °C under a load of 500 g is less than 100 µm, preferably less than 60 µm and more preferably less than 30 µm.

5. Pressure-sensitive adhesive tape according to at least one of the preceding claims, **characterized in that** the ratio of the microshear travels, µS2/µS1, is less than 0.3, preferably less than 0.2.

6. Pressure-sensitive adhesive tape according to at least one of the preceding claims, **characterized in that** the dynamic glass transition temperature of the pressure-sensitive adhesive at 10 rad/s is -10 °C to 15 °C and preferably -6 °C to 4 °C.

7. Pressure-sensitive adhesive tape according to at least one of the preceding claims, **characterized in that** the pressure-sensitive adhesive contains no additions at all of tackifier resins or plasticizers.

8. Pressure-sensitive adhesive tape according to at least one of the preceding claims, **characterized in that** the total thickness of the pressure-sensitive adhesive tapes without release film is 20 to 150 µm, preferably 50 to 100 µm.

9. Pressure-sensitive adhesive tape according to at least one of the preceding claims, **characterized in that** the bond strength to steel is at least 1.5 and preferably 2.5 N/cm and/or the bond strength to PET is at least 0.5 and preferably 1.0 N/cm.

10. Pressure-sensitive adhesive tape according to at least one of the preceding claims, **characterized in that** the carrier film is composed of PET.

11. Pressure-sensitive adhesive tape according to at least one of the preceding claims, **characterized in that** between the carrier material and the pressure-sensitive adhesives there is an adhesion promoter.

12. Use of a pressure-sensitive adhesive tape according to at least one of the preceding claims in medical diagnostic strips used to analyze biological fluids.

## Revendications

1. Bande autoadhésive pour bandes de diagnostic médical, permettant d'étudier des liquides biologiques, en un matériau de support revêtu sur une ou sur les deux faces à chaque fois d'un maximum de 20 g/m², de préférence d'un maximum de 15 g/m², d'une masse autoadhésive, la résistance au cisaillement de la masse autoadhésive à 25°C et 70°C et sous une charge pondérale de 1000 g étant de plus de 10 000 min., et le ou les polymères de la masse autoadhésive présentant une valeur K de plus de 55 Pa*s.

2. Bande autoadhésive suivant la revendication 1, **caractérisée en ce que** la masse autoadhésive consiste en un ou plusieurs copolymères, dans lesquels des monomères acryliques forment le principal constituant.

3. Bande autoadhésive suivant la revendication 1 ou 2, **caractérisée en ce que** les polymères de la masse autoadhésive ont une valeur K de plus de 60 Pa*s.

4. Bande autoadhésive suivant au moins l'une quelconque des revendications 1 à 3, **caractérisée en ce que** la course de microcisaillement de la bande autoadhésive après 15 min. à 40°C et sous une charge de 500 g est de moins de 100 µm, de préférence de moins de 60 µm et plus préférentiellement de moins de 30 µm.

5. Bande autoadhésive suivant au moins l'une quelconque des revendications qui précèdent, **caractérisée en ce que** le quotient de la course de microcisaillement µS2/µS1 est inférieur à 0,3, de préférence inférieur à 0,2.

6. Bande autoadhésive suivant au moins l'une quelconque des revendications qui précèdent, **caractérisée en ce que** la température de transition vitreuse dynamique de la masse autoadhésive à 10 rad/s est de -10°C à 15°C, de préférence de -6°C à 4°C.

7. Bande autoadhésive suivant au moins l'une quelconque des revendications qui précèdent, **caractérisée en ce que** la masse autoadhésive ne contient pas d'additions de résines donnant du collant ou de plastifiants.

8. Bande autoadhésive suivant au moins l'une quelconque des revendications qui précèdent, **caractérisée en ce que** l'épaisseur totale de la bande autoadhésive sans feuille de séparation est de 20 à 150 µm, de préférence de 50 à 100 µm.

9. Bande autoadhésive suivant au moins l'une quelconque des revendications qui précèdent, **caractérisée en ce que** la force d'adhérence sur l'acier est d'au moins 1,5 et de préférence de 2,5 N/cm et / ou que la force d'adhérence sur le PET est d'au moins 0,5 et de préférence de 1,0 N/cm.

10. Bande autoadhésive suivant au moins l'une quelconque des revendications qui précèdent, **caractérisée en ce que** la feuille de support est en PET.

11. Bande autoadhésive suivant au moins l'une quelconque des revendications qui précèdent, **caractérisée en ce qu'**un agent adhésif se trouve entre le matériau de support et la masse autoadhésive.

12. Utilisation d'une bande autoadhésive suivant au moins l'une quelconque des revendications qui précèdent dans des bandes de test médical diagnostique au moyen desquelles on étudie des liquides biologiques.
